# EUROPÄISCHE PATENTSCHRIFT

(11) **EP 2 843 778 B1**
(45) Veröffentlichungstag und Bekanntmachung des Hinweises auf die Patenterteilung: **27.06.2018**
(21) Anmeldenummer: 13004306.0
(22) Anmeldetag: 02.09.2013
(51) Int. Cl.: H01R 39/58, H01R 39/08

(54) **Schleifringeinheit und Verfahren zur Zustandsüberwachung einer Schleifringeinheit**
Slip ring unit and method for monitoring the state of a slip ring unit
Unité de bague collectrice et procédé de surveillance de l'état d'une unité de bague collectrice

(43) Veröffentlichungstag der Anmeldung: 04.03.2015
(73) Patentinhaber: LTN Servotechnik GmbH, 83624 Otterfing (DE); Leine Linde Systems GmbH, 20457 Hamburg (DE)
(72) Erfinder: Angerpointner, Ludwig, 81247 München (DE); Schmid, Gerhard, 21107 Hamburg (DE)
(74) Vertreter: Hofmann, Ernst

(56) Entgegenhaltungen:
- WO-A1-2010/000350
- JP-A- 2012 124 047
- SU-A1- 1 328 867
- SU-A1- 1 488 902
- US-A- 5 402 461
- US-A- 6 034 531

## Beschreibung

### GEBIET DER TECHNIK

Die Erfindung betrifft eine Schleifringeinheit zum Übertragen eines elektrischen Stroms zwischen zwei relativ zueinander drehbaren Bauteilgruppen, gemäß dem Anspruch 1 sowie ein Verfahren zur Zustandsüberwachung einer derartigen Schteifringeinheit, insbesondere für Windkraftanlagen, gemäß dem Anspruch 9.

Schleifringeinheiten bestehen üblicherweise aus zwei Bauteilgruppen, nämlich einem Stator und einem Rotor. Der Stator umfasst häufig Bürsten, wogegen der Rotor meist eine Folge von Schleifringen aufweist. Im Betrieb haben die Bürsten gleitenden Kontakt zu rotierenden Schleifringen entlang so genannter Spuren. Derartige Schleifringeinheiten werden in vielen technischen Gebieten eingesetzt, um elektrische Signale oder elektrische Leistung von einer ortsfesten auf eine sich drehende elektrische Einheit zu übertragen.

Beispielsweise werden derartige Schleifringeinheiten in der Rotornabe oder der Gondel von Windkraftanlagen eingebaut, etwa zur Übertragung von Steuersignalen und / oder elektrischen Strömen für Antriebe zur Verstellung der Anstellwinkel der Rotorblätter.

Gerade bei derart hochwertigen Anlagen oder Maschinen ist es wichtig, dass deren Verfügbarkeit nicht durch die Schleifringeinheit reduziert wird.

### STAND DER TECHNIK

In der Patentschrift US 6 034 531 ist eine Anordnung zur Verschleißüberwachung eines schleifenden elektrischen Kontakts für eine Rotorblattheizung eines Helikopters offenbart.

### ZUSAMMENFASSUNG DER ERFINDUNG

Der Erfindung liegt die Aufgabe zugrunde, eine Schleifringeinheit zu schaffen, deren Zustand auf einfache und wirtschaftliche Weise überwacht werden kann.

Diese Aufgabe wird erfindungsgemäß durch die Merkmale des Anspruchs 1 bzw. 9 gelöst.

Die Schleifringeinheit zum Übertragen eines elektrischen Stroms umfasst eine erste und eine zweite Bauteilgruppe, wobei die beiden Bauteilgruppen relativ zueinander um eine Achse drehbar angeordnet beziehungsweise gelagert sind. Weiterhin weist die Schleifringeinheit zumindest eine erste und eine zweite Spur auf. Die Schleifringeinheit ist so konfiguriert, dass über unterschiedliche Drehstellungen hinweg sich eine Bürste und ein Schleifring entlang einer der Spuren berühren. Die erste Bauteilgruppe weist einen ersten elektrischen Anschluss und eine elektrische Schaltung für eine Zustandsüberwachung auf. Die zweite Bauteilgruppe weist einen zweiten elektrischen Anschluss auf. Dabei ist der elektrische Strom vom ersten Anschluss zum zweiten Anschluss über die erste Spur übertragbar beziehungsweise fließt der Strom über die erste Spur. Ferner ist eine elektrische Spannung über die zweite Spur zur Schaltung übertragbar. Die Schleifringeinheit ist so konfiguriert, dass die erste Spur und die zweite Spur elektrisch verbunden sind.

Die Schleifringeinheit ist also zum Übertragen des elektrischen Stroms vom ersten zum zweiten Anschluss über die erste Spur konfiguriert und zum Übertragen der elektrischen Spannung über die zweite Spur zur Schaltung.

Unter dem Begriff elektrischer Strom sind im Folgenden elektrische Signale (also Strom mit minimaler Stromstärke) oder auch Strom zur Übertragung von elektrischer Leistung zu verstehen.

Als Anschlüsse können lösbare elektrische Verbindungen wie zum Beispiel Steckverbinder beziehungsweise elektrische Kupplungen oder Klemmverbindungen verwendet werden oder aber auch bedingt lösbare oder nicht lösbare elektrische Verbindungen, etwa Löt- oder Schweißanschlüsse.

Erfindungsgemäß weist die Schaltung eine Stromquelle zur Erzeugung eines Prüfstroms auf. Die Schleifringeinheit kann dann so konfiguriert sein, dass der Prüfstrom über die erste Spur und die zweite Spur fließt.

In Weiterbildung der Erfindung kann die Schaltung ein Mittel, etwa einen Schalter, insbesondere einen elektronischen Schalter beziehungsweise Halbleiterschalter, zum Unterbrechen des Prüfstroms aufweisen.

Die elektrische Verbindung der ersten Spur mit der zweiten Spur kann über einen Leiter realisiert werden oder dadurch, dass die Spuren auf ein und demselben leitenden Schleifring angeordnet sind. Mit Vorteil weist die elektrische Verbindung der ersten Spur mit der zweiten Spur einen geringen Widerstand auf, so dass die erste Spur und die zweite Spur praktisch das gleiche elektrische Potenzial aufweisen.

Eine Spur ist also eine Fläche, entlang welcher sich eine Bürste und ein Schleifring bei der relativen Drehung zwischen der ersten und der zweiten Bauteilgruppe berühren. Demnach wird entlang einer Spur bei der relativen Drehung schleifend kontinuierlich ein elektrischer Kontakt hergestellt. Insbesondere kann diese Fläche beziehungsweise die Spur umlaufend auf einer zylindrischen Mantelfläche eines Schleifrings angeordnet sein. Alternativ, beispielsweise bei so genannten Tellerschleifringen, kann die Spur umlaufend auf einer zylindrischen Stirnfläche verlaufen, beziehungsweise können mehrere Spuren konzentrisch zur Achse mit unterschiedlichen Durchmessern angeordnet sein.

Häufig wird die erste Bauteilgruppe als Stator eingesetzt, während die zweite Bauteilgruppe als Rotor arbeitet.

Die Schleifringeinheit kann Insbesondere so ausgestaltet sein, dass die erste Bauteilgruppe mehrere Bürsten und die zweite Bauteilgruppe zumindest einen Schleifring aus elektrisch leitendem Material, insbesondere Metall aufweist. In diesem Fall können die Spuren auf ein und demselben Schleifring angeordnet sein, der mit den mehreren Bürsten, insbesondere zwei Bürsten, in Berührkontakt ist.

Alternativ kann die Schleifringeinheit so ausgestaltet sein, dass die zweite Bauteilgruppe zumindest eine Bürste aus elektrisch leitendem Material und die erste Bauteilgruppe mehrere Schleifringe aufweist. In diesem Fall können die Spuren entlang ein und derselben Bürste verlaufen, welche zwei oder mehrere Schleifringe berührt.

Mit Vorteil sind sowohl die Bürste als auch der Schleifring aus Metall hergestellt, insbesondere aus Messing, Bronze, Edelstahl, einem Edelmetall oder Legierungen von Edelmetallen. Vorzugsweise weisen sowohl die Bürste als auch der Schleifring zumindest im Bereich der ersten Spur eine Silberlegierung auf.

Weiterhin können aber auch die Spuren auf unterschiedlichen Schleifringen angeordnet sein, welche durch einen Leiter elektrisch verbunden sind.

In weiterer Ausgestaltung der Erfindung kann die Schaltung für die Zustandsüberwachung ein Mittel zur Messung der über die zweite Spur übertragenen Spannung umfassen. Alternativ oder ergänzend kann die Schaltung ein Mittel zur Messung des über die erste Spur übertragenen Stroms und / oder des Prüfstroms umfassen.

In Weiterbildung der Erfindung kann die Schleifringeinheit mehrere Bürsten und mehrere Schleifringe und somit mehrere Spuren aufweisen, wobei über zumindest eine Spur keine elektrische Spannung zur Schaltung übertragbar ist. Wenngleich die Schleifringeinheit eine Vielzahl von Spuren beziehungsweise Schleifringen und Bürsten aufweisen kann, so dass eine Vielzahl von Strompfaden zwischen den Anschlüssen eingerichtet ist, brauchen nicht alle diese Strompfade durch die Zustandsüberwachung beobachtet werden. Üblicherweise reicht es aus, wenn ein ausgewählter Strompfad beziehungsweise Signalpfad beziehungsweise eine gewählte Spur entsprechend überwacht wird.

Mit Vorteil ist die Schleifringeinheit zum Zuführen von Messsignalen von Sensoren eines Drehgebers konfiguriert. Insbesondere kann der Drehgeber in die Schleifringeinheit integriert sein. Unter Drehgeber ist ein Messgerät zur Messung der relativen Drehstellung zwischen der ersten und der zweiten Bauteilgruppe zu verstehen. Der Drehgeber kann auch so ausgestaltet sein, dass dieser alternativ oder ergänzend zur aktuellen Drehstellung eine Anzahl der Umdrehungen zwischen der ersten und der zweiten Bauteilgruppe, die innerhalb eines bestimmten Zeitintervalls erfolgt sind, ausgibt. Die Schleifringeinheit kann so konfiguriert sein, dass die Messsignale des Drehgebers der Schaltung zur weiteren Auswertung im Zuge der Zustandsüberwachung zuführbar sind.

Die Schleifringeinheit kann insbesondere derart ausgestaltet sein, dass die Schleifringe in Richtung der Achse zueinander versetzt angeordnet sind.

Gemäß einem weiteren Aspekt betrifft die Erfindung ein Verfahren zur Zustandsüberwachung einer Schleifringeinheit. Dabei umfasst die Schleifringeinheit eine erste und eine zweite Bauteilgruppe, wobei die beiden Bauteilgruppen relativ zueinander um eine Achse drehbar angeordnet sind. Die Schleifringeinheit weist eine erste und eine zweite Spur auf und ist so konfiguriert, dass über unterschiedliche Drehstellungen hinweg sich Bürste und Schleifring entlang einer der Spuren berühren, so dass über die erste Spur ein elektrischer Strom von einem ersten Anschluss der ersten Bauteilgruppe zu einem zweiten Anschluss der zweite Bauteilgruppe übertragen wird. Dabei wird eine elektrische Spannung über die zweite Spur zur Schaltung übertragen. Durch die Schaltung wird eine Zustandsüberwachung in der Weise durchgeführt, dass basierend auf der Spannung ein Kennwert für den Zustand der Schleifringeinheit ermittelt wird.

Mit Vorteil wird die Höhe des Stroms, der über die erste Spur übertragen wird beziehungsweise über die erste Spur fließt, bestimmt beziehungsweise gemessen, wobei dieser Messwert der Schaltung zur Zustandsüberwachung zugeführt wird, so dass durch die Schaltung eine Zustandsüberwachung in der Weise durchgeführt wird, dass basierend auf der Spannung und des über die erste Spur übertragen Stroms ein Kennwert für den Zustand der Schleifringeinheit ermittelt wird.

Gemäß der Erfindung wird durch eine Stromquelle ein Prüfstrom erzeugt, der insbesondere nur zeitweise über die erste Spur und die zweite Spur geleitet wird, während ansonsten kein derartiger Prüfstrom (über die erste Spur und die zweite Spur) fließt.

Wenn der Prüfstrom nur zeitweise über die erste Spur und die zweite Spur geleitet wird, kann während dieser Zeit, also während der Prüfstrom über die erste Spur und die zweite Spur fließt, die Höhe des Stroms bestimmt werden.

Zudem kann durch die Schaltung eine Zustandsüberwachung in der Weise durchgeführt werden, dass basierend auf der Spannung, die über die zweite Spur zur Schaltung übertragen wird und des Stroms der zumindest über die erste Spur übertragen wird, ein Kennwert für den Zustand der Schleifringeinheit ermittelt wird. Insbesondere kann ein Übergangswiderstand und / oder ein Rauschpegel bei der Übertragung des Stromes bestimmt werden und als Kennwert dienen. Derartige Kennwerte beziehungsweise Parameter können Auskunft über den Zustand der Schleifringeinheit geben.

Weiterhin kann eine relative Drehstellung zwischen der ersten und der zweiten Bauteilgruppe oder die Anzahl der Umdrehungen zwischen der ersten und der zweiten Bauteilgruppe gemessen werden. Die daraus resultierenden Messsignale können dann der Schaltung zugeführt werden, so dass durch die Schaltung eine Zustandsüberwachung in der Weise durchgeführt wird, dass basierend auf der Spannung und den Messsignalen ein Kennwert für den Zustand der Schleifringeinheit ermittelt wird. Die Anzahl der Umdrehungen kann für einen definierten Zeitraum ermittelt werden, beispielsweise kann die Anzahl den Wert der gesamten Anzahl der Umdrehungen vom Betriebsbeginn der Schleifringeinheit bis zum jeweils aktuellen Zeitpunkt annehmen. Ebenso kann die Drehzahl beziehungsweise Drehgeschwindigkeit während der Messung bei der Bildung des Kennwerts beziehungsweise zur der Beurteilung des Zustands der Schleifringeinheit berücksichtigt werden.

In weiterer Ausgestaltung der Erfindung kann eine relative Feuchte gemessen werden, beispielsweise mit Hilfe eines Feuchtesensors. Der Messwert der relativen Feuchte wird der Schaltung zugeführt. Zudem kann in der Schleifringeinheit ein Temperatursensor angeordnet sein, so dass beispielsweise eine genaue Taupunktermittlung durchgeführt werden kann. Insbesondere zur Ursachenfindung von Störungen kann auch ein Vibrationssensor, dessen Signal der Schaltung für die Zustandsüberwachung zugeführt wird, in der Schleifringeinheit angeordnet sein.

Durch die Schaltung wird eine Zustandsüberwachung in der Weise durchgeführt, dass basierend auf der Spannung und der relativen Feuchte und / oder der Temperatur und / oder des ermittelten Taupunkts und / oder des Vibrationssensorsignals ein Kennwert für den Zustand der Schleifringeinheit ermittelt wird.

Insbesondere kann in der Schleifringeinheit ein Grenzwert hinterlegt sein, welcher durch die Schaltung mit einem Kennwert für den Zustand der Schleifringeinheit verglichen wird, wobei bei Überschreiten des Grenzwerts von der Schleifringeinheit eine Warnung ausgegeben wird.

Gemäß einem weiteren Aspekt der Erfindung kann die Schleifringeinheit in einer Gondel einer Windkraftanlage verwendet werden, insbesondere zur Übertragung von Steuersignalen und / oder Strömen für Elektroantriebe einer Rotorblattverstellung.

Vorteilhafte Ausbildungen der Erfindung entnimmt man den abhängigen Ansprüchen.

Weitere Einzelheiten und Vorteile der erfindungsgemäßen Schleifringeinheit ergeben sich aus der nachfolgenden Beschreibung von Ausführungsbeispielen anhand der beiliegenden Figuren.

### KURZE BESCHREIBUNG DER ZEICHNUNGEN

Es zeigen die
- Figur 1: eine Längsschnittdarstellung einer Schleifringeinheit,
- Figur 2a: eine schematische Ansicht von Elementen der Schleifringeinheit gemäß einem ersten Ausführungsbeispiel während des Normalbetriebs,
- Figur 2b: eine schematische Ansicht von Elementen der Schleifringeinheit gemäß einem ersten Ausführungsbeispiel während des Prüfbetriebs,
- Figur 3: eine schematische Ansicht von Elementen der Schleifringeinheit gemäß einem zweiten Ausführungsbeispiel während des Prüfbetriebs.

### BESCHREIBUNG DER AUSFÜHRUNGSFORMEN

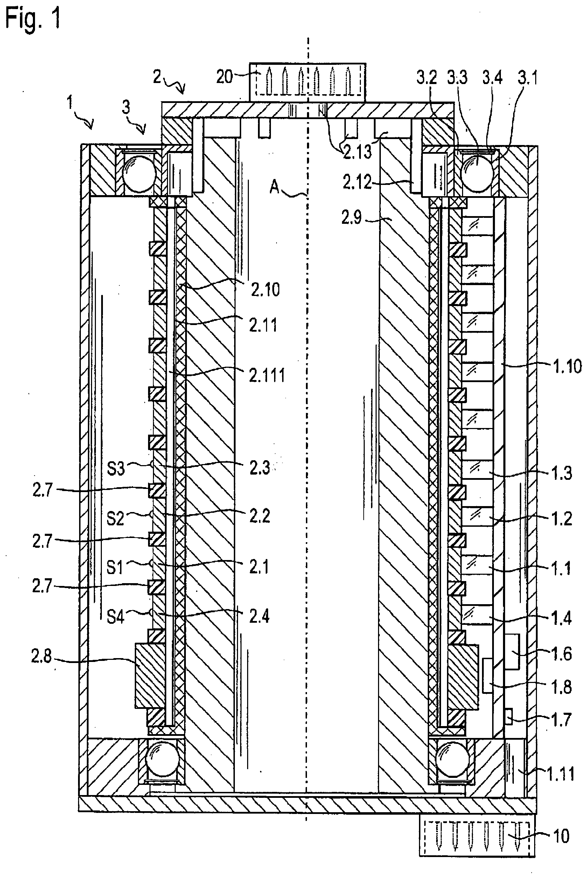

Gemäß der Figur 1 umfasst die Schleifringeinheit eine erste Bauteilgruppe 1, die als Stator bezeichnet werden kann und eine zweite Bauteilgruppe 2, die im Folgenden als Rotor fungiert. Die Schleifringeinheit dient zum mehrkanaligem Übertragen von elektrischen Strömen zwischen einem ersten elektrischen Anschluss 10 der ersten Bauteilgruppe 1 zu einem zweiten elektrischen Anschluss 20 der zweiten Bauteilgruppe 2. Die beiden Bauteilgruppen sind 1 relativ zueinander um eine Achse A drehbar angeordnet.

Die zweite Bauteilgruppe 2 umfasst eine Welle 2.9, die im vorliegenden Beispiel als Hohlwelle ausgestaltet ist Radial außerhalb der Welle 2.9 befindet sich zunächst ein im Wesentlichen rohrförmiges Bauteil 2.10, welches beispielsweise aus Aluminium hergestellt sein kann. Daran weiterhin radial außerhalb anschließend ist ein Trägerrohr 2.11 angeordnet, das in Axialrichtung verlaufende Ausnehmungen 2.111 aufweist. Am elektrisch isolierenden Trägerrohr 2.11, das vorzugsweise aus Kunststoff oder Keramik hergestellt sein kann, sind konzentrisch zur Achse A verdrehsicher Schleifringe 2.1 - 2.4 befestigt, die durch elektrisch isolierende Ringe 2.7 mit axialem Abstand voneinander getrennt sind. Der Übersichtlichkeit halber wurde in den Figuren auf die Darstellung von elektrischen Leitungen verzichtet. Dessen ungeachtet ist im Ausführungsbeispiel jeder der mit axialem Abstand aneinandergereihten Schleifringe 2.1 - 2.4 an seiner Innenseite mit einer elektrischen Leitung verbunden, die jeweils längs einer Ausnehmung 2.111 in Axialrichtung in der zweiten Bauteilgruppe 2 und weiterhin durch eine der Rillen 2.12 und Bohrungen 1.13 verlegt ist. Demgemäß sind die elektrischen Leitungen mit Leitern eines elektrischen Anschlusses 20, der hier als Steckverbinder ausgestaltet ist und an einem axialen Ende platziert ist, kontaktiert.

Neben den Schleifringen 2.1 - 2.4 ist an dem Trägerrohr 2.11 drehfest eine Maßverkörperung 2.8 montiert, welche im vorgestellten Ausführungsbeispiel im Wesentlichen ringförmig ausgestaltet ist. Im gezeigten Ausführungsbeispiel weist die Maßverkörperung 2.8 an ihrer radial außen liegenden Mantelseite eine magnetische Teilung beziehungsweise Skalierung auf. Die Anordnung ist so ausgestaltet, dass die Maßverkörperung 2.8 in die Folge der Schleifringe 2.1 - 2.4 eingereiht ist und somit konzentrisch zur Achse A mit axialem Versatz zu den Schleifringen 2.1 - 2.4 montiert ist.

Die erste Bauteilgruppe 1 umfasst den elektrischen Anschluss 10, der hier in Form einer Steckkupplung ausgebildet ist. Der Anschluss 10 (Steckkupplung) weist mehrere in der Figur 1 gestrichelt dargestellte Leiterpins auf, die mit Kabeln verbunden sind. Der Übersichtlichkeit halber sind in der Figur 1 die Kabel selbst nicht dargestellt. Die Kabel werden durch eine Bohrung 1.11 ins Innere der ersten Bauteilgruppe 1 geführt und sind mit Leitern, die auf einem Halter 1.10 aus einem isolierenden Werkstoff fixiert sind, elektrisch verbunden.

An dem Halter 1.10 sind Bürsten 1.1 - 1.4 befestigt, welche der ersten Bauteilgruppe 1 zuzuordnen sind und federnd die Schleifringe 2.1 - 2.4 berühren. Die Bürsten 1.1 - 1.4 sind aus Metall, insbesondere aus einer Silberlegierung, hergestellt.

Weiterhin befindet sich auf dem Halter 1.10 eine elektrische Schaltung 1.6 zur Zustandsüberwachung sowie ein Abtastkopf 1.8, der zur Abtastung der Maßverkörperung 2.8, beispielsweise mit Hilfe magnetoresistiver Sensoren, geeignet ist. Zudem ist am Halter 1.10 ein weiterer Sensor 1.7 angeordnet, der hier als Feuchtesensor ausgebildet ist.

Die erste Bauteilgruppe 1 und die zweite Bauteilgruppe 2 sind mit Hilfe von Wälzlagern 3 relativ zueinander drehbar angeordnet. Die Wälzlager 3 umfassen einen Außenring 3.1, einen Innenring 3.2 sowie Wälzkörper 3.3 und eine, insbesondere schleifende, Dichtung 3.4.

Die Schleifringeinheit kann gemäß dem Ausführungsbeispiel in einer Gondel einer Windkraftanlage montiert werden und zur Übertragung elektrischer Ströme S im Sinne von Signalen zum Beispiel in einem Bus-System zwischen einer zentralen Steuerungseinheit und einem rotorseitigen Gerät X, beispielsweise ein Umrichter, verwendet werden. Die Signale werden etwa zur Ansteuerung von Elektroantrieben bzw. Elektromotoren, die zur Verstellung der Anstellwinkel der Rotorblätter dienen, benötigt. Weiterhin kann ein elektrischer Strom zur Energieversorgung über die Schleifringeinheit zu Elektroantrieben bzw. Elektromotoren, die zur Verstellung der Anstellwinkel der Rotorblätter dienen, übertragen werden. Derartige Antriebsmotoren sind in der Rotornabe der Windkraftanlage montiert und drehen sich folglich mit dem Windkraftanlagenrotor. Die erste Bauteilgruppe 1 der Schleifringeinheit ist also verdrehfest mit der Gondel verbunden.

Da mit einem Ausfall der Schleifringeinheit ein Ausfall der gesamten Windkraftanlage einhergeht, ist es wichtig den Zustand der Schleifringeinheit zu überwachen, so dass diese im Bedarfsfall während eines planmäßigen Wartungsintervalls repariert beziehungsweise ausgetauscht werden kann.

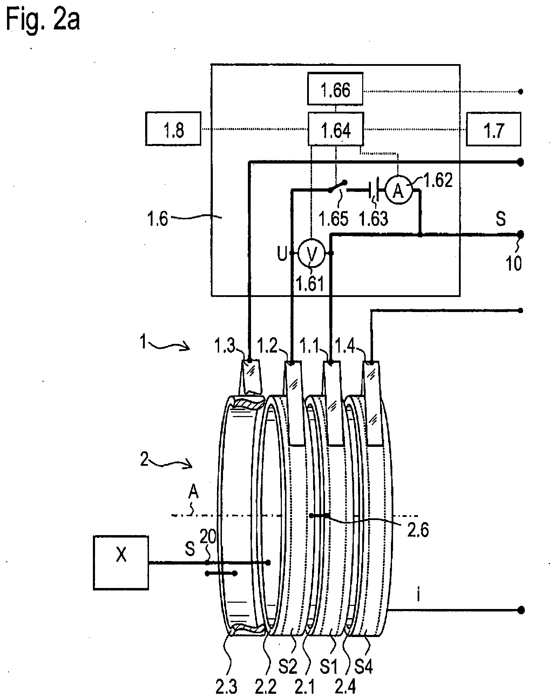

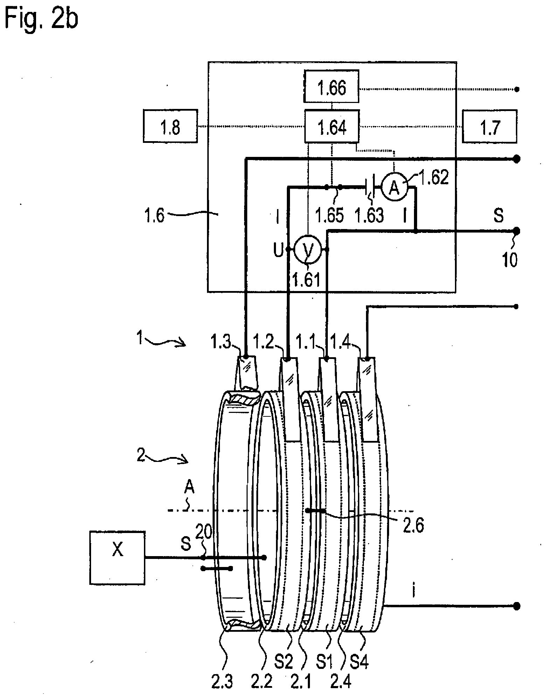

In den Figuren 2a, 2b ist beispielhaft ein vereinfachter Ausschnitt der Schleifringeinheit gezeigt, wobei unter anderem der Übersichtlichkeit halber auf die Darstellung der isolierenden Ringe 2.7 verzichtet wurde. Die Schleifringeinheit ist so konfiguriert, dass sich in unterschiedlichen Drehstellungen jeweils eine der Bürsten 1.1 - 1.4 und ein Schleifring 2.1 - 2.4 entlang so genannter Spuren S1, S2, S3, S4 berühren. Als Spur ist also hier ein Ausschnitt aus der Mantelfläche eines Schleifrings 2.1 - 2.4 zu verstehen, der in der Figur 2 axial zwischen den gestrichelten Linien angeordnet ist. Jeweils eine der Bürsten 1.1 - 1.4 berührt in unterschiedlichen Drehstellungen, z. B. während einer Umdrehung, den zugehörigen Schleifring 2.1 - 2.4 entlang einer dieser Spuren S1, S2, S3, S4. Im vorgestellten Ausführungsbeispiel weisen die Schleifringe 2.1 - 2.4 im Bereich der Spuren S1, S2, S3, S4 eine Silberlegierung auf.

Zwei Schleifringe 2.1, 2.2 sind durch einen Leiter 2.6 elektrisch verbunden, so dass diese Schleifringe 2.1, 2.2 das gleiche elektrische Potenzial aufweisen beziehungsweise kurzgeschlossen sind.

Der elektrische Strom S wird vom ersten Anschluss 10 zum zweiten Anschluss 20 über die erste Spur S1 mit Hilfe der Bürste 1.1 und des Schleifrings 2.1 übertragen. Weiterhin wird z. B. über eine weitere Bürste 1.4 und über einen anderen Schleifring 2.4 ein Strom i zu einem anderen Verbraucher übertragen.

Im Betrieb der Windkraftanlage wird z. B. von einer statorseitigen Steuerung ein Signal beziehungsweise ein Strom S über den Anschluss 10 in die Schleifringeinheit eingeleitet und durch die Bürste 1.1 entlang der Spur S1 auf den Schleifring 2.1 schleifend übertragen. Über den Leiter 2.6 fließt der Strom S zum benachbarten Schleifring 2.2. Dieser ist mit dem zweiten Anschluss 20, der an der zweiten Bauteilgruppe 2 angeordnet ist, elektrisch verbunden. Mit diesem zweiten Anschluss 20 ist zudem das elektrische Gerät X verbunden.

Um eine Information über den Zustand der Schleifringeinheit zu gewinnen, wird in vorgebenden Zeitabständen (beispielsweise einmal pro Tag) oder nach einer bestimmten Anzahl von Umdrehungen, kurzzeitig ein Prüfstrom I über die Spur S1 von der Bürste 1.1 zum Schleifring 2.1 übertragen (Figur 2b). Da die Schleifringe 2.1, 2.2 durch den Leiter 2.6 elektrisch verbunden sind, fließt der Prüfstrom I vom Schleifring 2.2 zur Bürste 1.2. Zu diesem Zweck ist eine Stromquelle 1.63 vorgesehen, wobei durch ein Schaltmittel 1.65 der betreffende Stromkreis, ausgelöst durch ein Signal der Recheneinheit 1.64, geschlossen wird. Die Stromquelle 1.63 liefert Gleichstrom bei vergleichsweise geringer Spannung, da die Übergangswiderstände sehr klein sind. Beispielsweise kann als Stromquelle 1.63 eine Batterie (vorzugsweise ein aufladbare Batterie) zusammen mit einem Widerstand zur Begrenzung des Prüfstroms I verwendet werden.

Entlang der Spur S2 wird also vom Schleifring 2.2 über die Bürste 1.2 eine elektrische Verbindung zur Schaltung 1.6 hergestellt, so dass eine Spannung U über die zweite Spur S2 vom Schleifring 2.2 über die Bürste 1.2 zur Schaltung 1.6 übertragbar ist. Die anliegende Spannung U zwischen den Bürsten 1.1 und 1.2 wird durch ein zweites Messmittel 1.61 bestimmt und der Recheneinheit 1.64 zugeführt. Zudem wird durch ein zweites Messmittel 1.62 die Höhe des Stroms I bestimmt, wobei dann dieser Wert einer Recheneinheit 1.64 zugeführt wird.

Außerdem wird der Recheneinheit 1.64 die mit Hilfe des Abtastkopfes 1.8 gewonnene Information über die Ist-Drehzahl und die Anzahl der bereits von der Schleifringeinheit geleisteten Umdrehungen zugeführt. Die Information über die relative Feuchte, die der Sensor 1.7 liefert, wird ebenso an die Recheneinheit 1.64 weitergegeben.

In der Recheneinheit 1.64 wird zunächst basierend auf der gemessenen Höhe des Stroms I und der Spannung U ein Übergangswiderstand als Kennwert für den Zustand der Schleifringeinheit ermittelt. Gerade in der ersten Betriebsphase oder Einlaufphase der Schleifringeinheit kann dieser Übergangswiderstand vergleichsweise hoch sein. Im weiteren Betrieb der Schleifringeinheit fällt aber der Übergangswiderstand erfahrungsgemäß auf ein Normalmaß zurück. Durch Heranziehen der bereits geleisteten Umdrehungen, kann beispielsweise festgestellt werden, dass sich die Schleifringeinheit noch in der Einlaufphase befindet, so dass keine Warnung ausgegeben werden muss. Gleichermaßen kann zur Beurteilung des Übergangswiderstandes die aktuell gemessene relative Feuchte der Luft in der Schleifringeinheit herangezogen werden, um festzulegen, ob der gemessene Übergangswiderstand eine Warnung auslösen soll oder nicht. Somit kann also durch Heranziehen der geleisteten Umdrehungen und / oder der relativen Feuchte der Luft ein verfeinerter Kennwert für den Zustand der Schleifringeinheit ermittelt werden. Der Kennwert wird dann in einem Speicher 1.66 abgelegt und kann jederzeit über eine entsprechende Schnittstelle von einer Folgeelektronik ausgelesen werden.

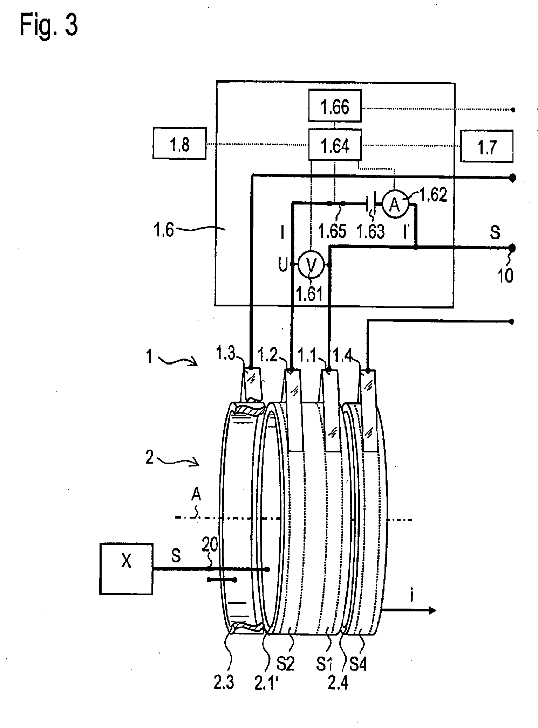

Gemäß einem zweiten Ausführungsbeispiel nach der Figur 3 umfasst die Schleifringeinheit einen Schleifring 2.1', der in Axialrichtung vergleichsweise breit ausgestaltet ist, so dass zwei Bürsten 1.1, 1.2 entlang zwei axial beabstandeten Spuren S1 und S1 den Schleifring 2.1' kontaktieren. Auf dem Schleifring 2.1' sind also zwei Spuren S1, S1. Der Schleifring 2.1' ist aus elektrisch leitendem Metall hergestellt und weist entlang der Spuren S1, S2 eine Beschichtung mit einem Silberanteil auf. Da der Schleifring 2.1' elektrisch leitend ist, kann auf einen separaten Leiter zum elektrischen Verbinden der ersten Spur S1 mit der zweiten Spur S2 verzichtet werden. Bei dieser Konfiguration wird durch die Bürste 1.1 der Strom I entlang der Spur S1 auf den Schleifring 2.1' schleifend übertragen. Der Schleifring 2.1' ist mit dem zweiten Anschluss 20, welcher an der zweiten Bauteilgruppe 2 angeordnet ist, elektrisch verbunden ist. Mit diesem zweiten Anschluss 20 ist wiederum das elektrische Gerät X verbunden. Über die zweite Spur S2 auf dem Schleifring 2.1' ist eine elektrische Spannung U vom Schleifring 2.1' über die Bürste 2.1 zur Schaltung 1.6 übertragbar.

In der Schaltung 1.6 kann nun entsprechend dem ersten Ausführungsbeispiel eine Zustandsüberwachung durchgeführt werden, so dass basierend auf der Spannung U ein Kennwert für den Zustand der Schleifringeinheit gegebenenfalls unter Einbeziehung der geleisteten Umdrehung oder der relativen Feuchte der Luft in der Schleifringeinheit ermittelt wird.

## Patentansprüche

1. Schleifringeinheit zur Übertragung eines elektrischen Stroms (S) umfassend eine erste und eine zweite Bauteilgruppe (1, 2), wobei die beiden Bauteilgruppen (1, 2) relativ zueinander um eine Achse (A) drehbar angeordnet sind und die Schleifringeinheit eine erste und eine zweite Bürste (1.1, 1.2) sowie eine erste und eine zweite Spur (S1, S2) aufweist und die Schleifringeinheit so konfiguriert ist, dass sich über unterschiedliche Drehstellungen hinweg die erste Bürste (1.1) und ein Schleifring (2.1; 2.1') entlang derersten Spur (S1) berühren und die zweite Bürste (1.2) und ein Schleifring (2.2; 2.1') sich entlang der zweiten Spur (S2) berühren, so dass jeweils entlang einer der Spuren (S1, S2) bei einer relativen Drehung schleifend kontinuierlich ein elektrischer Kontakt herstellbar ist, wobei
- die erste Bauteilgruppe (1) die erste und die zweite Bürste (1.1, 1.2), einen ersten Anschluss (10) und eine elektrische Schaltung (1.6) für eine Zustandsüberwachung aufweist, und
- die zweite Bauteilgruppe (2) den Schleifring (2.1, 2.2; 2.1') und einen zweiten Anschluss (20) aufweist, wobei
der elektrische Strom (S) vom ersten Anschluss (10) zum zweiten Anschluss (20) über den Kontakt zwischen erster Bürste (1.1) und erster Spur (S1) übertragbar ist,
eine elektrische Spannung (U) über den Kontakt zwischen zweiter Spur (S2) und zweiter Bürste (1.2) zur Schaltung (1.6) übertragbar ist, und
die Schleifringeinheit so konfiguriert ist, dass die erste Spur (S1) und die zweite Spur (S2) elektrisch verbunden sind,
**dadurch gekennzeichnet, dass**
die Schaltung (1.6) eine Stromquelle (1.63) zur Erzeugung eines Prüfstroms (I) und ein Schaltmittel (1.65) aufweist und die Schleifringeinheit so konfiguriert ist, dass der Prüfstrom (I) über den Kontakt zwischen erster Bürste (1.1) und erster Spur (S1), eine elektrische Verbindung zwischen erster Spur (S1) und zweiter Spur (S2) und den Kontakt zwischen zweiter Spur (S2) und zweiter Bürste (1.2) fließt, wobei dieser Stromkreis durch das Schaltmittel (1.65), ausgelöst durch ein Signal einer Recheneinheit (1.64), schließbar ist

2. Schleifringeinheit gemäß dem Anspruch 1, wobei die erste Bauteilgruppe (1) mehrere Bürsten (1.1, 1.2) und die zweite Bauteilgruppe (2) zumindest einen Schleifring (2.1') aufweist.

3. Schleifringeinheit gemäß dem Anspruch 2, wobei die Spuren (S1, S2) auf ein und demselben Schleifring (2.1') angeordnet sind.

4. Schleifringeinheit gemäß einem der Ansprüche 1 bis2, wobei die Spuren (S1, S2) auf unterschiedlichen Schleifringen (2.1 - 2.5) angeordnet sind, welche durch einen Leiter (2.6) elektrisch verbunden sind.

5. Schleifringeinheit gemäß einem der vorhergehenden Ansprüche, wobei die Schaltung (1.6) ein Mittel (1.61) zur Messung der Spannung (U) umfasst.

6. Schleifringeinheit gemäß einem der vorhergehenden Ansprüche, wobei die Schaltung (1.6) ein Mittel (1.62) zur Messung des Stromes (S) und / oder des Prüfstroms (I) umfasst.

7. Schleifringeinheit gemäß einem der vorhergehenden Ansprüche, wobei die Schleifringeinheit so konfiguriert ist, dass der Schaltung (1.6) Messsignale von Sensoren (1.8) eines Drehgebers (1.8, 2.8) zuführbar sind.

8. Schleifringeinheit gemäß einem der vorhergehenden Ansprüche, wobei die Schleifringeinheit einen Drehgeber (1.8, 2.8) aufweist zur Messung der relativen Drehstellung zwischen der ersten und der zweiten Bauteilgruppe (1, 2) oder der Anzahl der Umdrehungen zwischen der ersten und der zweiten Bauteilgruppe (1, 2), wobei die Messsignale des Drehgebers (1.8, 2.8) der Schaltung (1.6) zuführbar sind.

9. Verfahren zur Zustandsüberwachung einer Schleifringeinheit, welche gemäß dem Anspruch 1 ausgestaltet ist, wobei
- ein elektrischer Strom (S) von dem ersten Anschluss (10) der ersten Bauteilgruppe (1) zu dem zweiten Stromanschluss (20) der zweiten Bauteilgruppe (2) übertragen wird, wobei
- eine elektrische Spannung (U) über die zweite Spur (S2) zur Schaltung (1.6) übertragen wird und
- durch die Schaltung (1.6) eine Zustandsüberwachung in der Weise durchgeführt wird, dass basierend auf der Spannung (U) ein Kennwert für den Zustand der Schleifringeinheit ermittelt wird.

10. Verfahren gemäß dem Anspruch 9, wobei
- die Höhe des Stroms (S) bestimmt wird und dieser Messwert der Schaltung (1.6) zugeführt wird und
- durch die Schaltung (1.6) eine Zustandsüberwachung in der Weise durchgeführt wird, dass basierend auf der Spannung (U) und dem Strom (S) ein Kennwert für den Zustand der Schleifringeinheit ermittelt wird.

11. Verfahren gemäß dem Anspruch 9 oder 10, wobei der Prüfstrom (I) nur zeitweise über die erste Spur (S1) und die zweite Spur (S2) geleitet wird.

12. Verfahren gemäß dem Anspruch 9, 10 oder 11 wobei der Prüfstrom (I) nur zeitweise über die erste Spur (S1) und die zweite Spur (S2) geleitet wird und während dieser Zeit die Höhe des Stroms (S) bestimmt wird.

13. Verfahren zur Zustandsüberwachung einer Schleifringeinheit gemäß einem der Ansprüche 9 bis 12, wobei
- eine relative Drehstellung zwischen der ersten und der zweiten Bauteilgruppe (1, 2) oder die Anzahl der Umdrehungen zwischen der ersten und der zweiten Bauteilgruppe (1, 2) gemessen wird und die daraus resultierenden Messsignale der Schaltung (1.6) zugeführt werden und
- durch die Schaltung (1.6) eine Zustandsüberwachung in der Weise durchgeführt wird, dass basierend auf der Spannung (U) und den Messsignalen ein Kennwert für den Zustand der Schleifringeinheit ermittelt wird.

14. Verfahren zur Zustandsüberwachung einer Schleifringeinheit gemäß einem der Ansprüche 9 bis 13, wobei
- eine relative Feuchte gemessen wird und der Messwert der relativen Feuchte der Schaltung (1.6) zugeführt wird und
- durch die Schaltung (1.6) eine Zustandsüberwachung in der Weise durchgeführt wird, dass basierend auf der Spannung (U) und der relativen Feuchte ein Kennwert für den Zustand der Schleifringeinheit ermittelt wird.

15. Verfahren zur Zustandsüberwachung einer Schleifringeinheit gemäß einem der Ansprüche 9 bis 14, wobei in der Schleifringeinheit ein Grenzwert hinterlegt ist, welcher durch die Schaltung (1.6) mit einem Kennwert für den Zustand der Schleifringeinheit verglichen wird, wobei bei Überschreiten des Grenzwerts von der Schleifringeinheit eine Warnung ausgegeben wird.

## Claims

1. Slip-ring unit for transmitting an electric current (S) comprising a first and a second component group (1, 2), wherein the two component groups (1, 2) are arranged such that they can rotate relative to one another about an axis (A) and the slip-ring unit has a first and a second brush (1.1, 1.2) and a first and a second track (S1, S2) and the slip-ring unit is configured in such a way that the first brush (1.1) and a slip ring (2.1; 2.1') are in contact along the first track (S1) across different rotational positions and the second brush (1.2) and a slip ring (2.2; 2.1') are in contact along the second track (S2), so that an electrical contact can be established continuously in a sliding manner in each case along one of the tracks (S1, S2) in the case of a relative rotation, wherein
- the first component group (1) has the first and the second brush (1.1, 1.2), a first connection (10) and an electrical circuit (1.6) for state monitoring, and
- the second component group (2) has the slip ring (2.1, 2.2; 2.1') and a second connection (20), wherein
the electric current (S) can be transmitted from the first connection (10) to the second connection (20) via the contact between the first brush (1.1) and the first track (S1),
an electrical voltage (U) can be transmitted to the circuit (1.6) via the contact between the second track (S2) and the second brush (1.2), and
the slip-ring unit is configured in such a way that the first track (S1) and the second track (S2) are electrically connected,
**characterized in that**
the circuit (1.6) has a current source (1.63) for generating a test current (I) and a switching means (1.65) and the slip-ring unit is configured in such a way that the test current (I) flows via the contact between the first brush (1.1) and the first track (S1), an electrical connection between the first track (S1) and the second track (S2) and the contact between the second track (S2) and the second brush (1.2), wherein this current circuit can be closed by the switching means (1.65), triggered by a signal of a computation unit (1.64).

2. Slip-ring unit according to Claim 1, wherein the first component group (1) has a plurality of brushes (1.1, 1.2) and the second component group (2) has at least one slip ring (2.1').

3. Slip-ring unit according to Claim 2, wherein the tracks (S1, S2) are arranged on one and the same slip ring (2.1').

4. Slip-ring unit according to either of Claims 1 and 2, wherein the tracks (S1, S2) are arranged on different slip rings (2.1-2.5), which are electrically connected by a conductor (2.6).

5. Slip-ring unit according to one of the preceding claims, wherein the circuit (1.6) comprises a means (1.61) for measuring the voltage (U).

6. Slip-ring unit according to one of the preceding claims, wherein the circuit (1.6) comprises a means (1.62) for measuring the current (S) and/or the test current (I).

7. Slip-ring unit according to one of the preceding claims, wherein the slip-ring unit is configured in such a way that measurement signals from sensors (1.8) of a rotary encoder (1.8, 2.8) can be fed to the circuit (1.6).

8. Slip-ring unit according to one of the preceding claims, wherein the slip-ring unit has a rotary encoder (1.8, 2.8) for measuring the relative rotational position between the first and the second component group (1, 2) or the number of rotations between the first and the second component group (1, 2), wherein the measurement signals of the rotary encoder (1.8, 2.8) can be fed to the circuit (1.6).

9. Method for monitoring the state of a slip-ring unit, which is designed according to Claim 1, wherein
- an electric current (S) is transmitted from the first connection (10) of the first component group (1) to the second current connection (20) of the second component group (2), wherein
- an electrical voltage (U) is transmitted to the circuit (1.6) via the second track (S2) and
- the state is monitored by the circuit (1.6) in such a way that a characteristic value for the state of the slip-ring unit is identified based on the voltage (U).

10. Method according to Claim 9, wherein
- the magnitude of the current (S) is determined and this measurement value is fed to the circuit (1.6) and
- the state is monitored by the circuit (1.6) in such a way that a characteristic value for the state of the slip-ring unit is identified based on the voltage (U) and the current (S).

11. Method according to Claim 9 or 10, wherein the test current (I) is conducted only temporarily via the first track (S1) and the second track (S2).

12. Method according to Claim 9, 10 or 11, wherein the test current (I) is conducted only temporarily via the first track (S1) and the second track (S2) and the magnitude of the current (S) is determined during this time.

13. Method for monitoring the state of a slip-ring unit according to one of Claims 9 to 12, wherein
- a relative rotational position between the first and the second component group (1, 2) or the number of rotations between the first and the second component group (1, 2) is measured and the resulting measurement signals are fed to the circuit (1.6) and
- the state is monitored by the circuit (1.6) in such a way that a characteristic value for the state of the slip-ring unit is identified based on the voltage (U) and the measurement signals.

14. Method for monitoring the state of a slip-ring unit according to one of Claims 9 to 13, wherein
- a relative humidity is measured and the measurement value of the relative humidity is fed to the circuit (1.6) and
- the state is monitored by the circuit (1.6) in such a way that a characteristic value for the state of the slip-ring unit is identified based on the voltage (U) and the relative humidity.

15. Method for monitoring the state of a slip-ring unit according to one of Claims 9 to 14, wherein a limit value is stored in the slip-ring unit, said limit value being compared with a characteristic value for the state of the slip-ring unit by the circuit (1.6), wherein a warning is output by the slip-ring unit when the limit value is exceeded.

## Revendications

1. Unité de bague collectrice pour la transmission d'un courant électrique (S) comprenant un premier et un second groupes de composants (1, 2), dans laquelle les deux groupes de composants (1, 2) sont disposés de façon rotative l'un par rapport à l'autre autour d'un axe (A) et l'unité de bague collectrice présente un premier et un second balais (1.1, 1.2) ainsi qu'une première et une seconde pistes (S1, S2) et l'unité de bague collectrice est configurée de telle manière que tout au long de différentes positions de rotation le premier balai (1.1) et une bague collectrice (2.1; 2.1') se touchent le long de la première piste (S1) et que le second balai (1.2) et une bague collectrice (2.2; 2.1') se touchent le long de la seconde piste (S2), de telle manière que chaque fois le long d'une des pistes (S1, S2) lors d'une rotation relative un contact électrique glissant soit réalisable en continu, dans laquelle
- le premier groupe de composants (1) présente le premier et le second balais (1.1, 1.2), un premier raccord (10) et un circuit électrique (1.6) pour une surveillance d'état, et
- le second groupe de composants (2) présente la bague collectrice (2.1, 2.2; 2.1') et un second raccord (20), dans laquelle
le courant électrique (S) peut être transmis du premier raccord (10) au second raccord (20) par le contact entre le premier balai (1.1) et la première piste (S1),
une tension électrique (U) peut être transmise au circuit (1.6) par le contact entre la seconde piste (S2) et le second balai (1.2), et
l'unité de bague collectrice est configurée de telle manière que la première piste (S1) et la seconde piste (S2) soient électriquement reliées,
**caractérisée en ce que**
le circuit (1.6) présente une source de courant (1.63) pour la production d'un courant de contrôle (I) et un moyen de commutation (1.65) et l'unité de bague collectrice est configurée de telle manière que le courant de contrôle (I) circule par le contact entre le premier balai (1.1) et la première piste (S1), une liaison électrique entre la première piste (S1) et la seconde piste (S2) et le contact entre la seconde piste (S2) et le second balai (1.2), dans lequel ce circuit électrique peut être fermé par le moyen de commutation (1.65), déclenché par un signal d'une unité de calcul (1.64).

2. Unité de bague collectrice selon la revendication 1, dans laquelle le premier groupe de composants (1) présente plusieurs balais (1.1, 1.2) et le second groupe de composants (2) présente au moins une bague collectrice (2.1').

3. Unité de bague collectrice selon la revendication 2, dans laquelle les pistes (S1, S2) sont disposées sur une seule et même bague collectrice (2.1').

4. Unité de bague collectrice selon une des revendications 1 à 2, dans laquelle les pistes (S1, S2) sont disposées sur des bagues collectrices différentes (2.1 - 2.5), qui sont électriquement reliées au moyen d'un conducteur (2.6).

5. Unité de bague collectrice selon l'une quelconque des revendications précédentes, dans laquelle le circuit (1.6) comprend un moyen (1.61) pour la mesure de la tension (U).

6. Unité de bague collectrice selon l'une quelconque des revendications précédentes, dans laquelle le circuit (1.6) comprend un moyen (1.62) pour la mesure du courant (S) et/ou du courant de contrôle (I).

7. Unité de bague collectrice selon l'une quelconque des revendications précédentes, dans laquelle l'unité de bague collectrice est configurée de telle manière que des signaux de mesure de capteurs (1.8) d'un capteur rotatif (1.8, 2.8) puissent être envoyés au circuit (1.6).

8. Unité de bague collectrice selon l'une quelconque des revendications précédentes, dans laquelle l'unité de bague collectrice présente un capteur rotatif (1.8, 2.8) pour la mesure de la position de rotation relative entre le premier et le second groupes de composants (1, 2) ou du nombre de révolutions entre le premier et le second groupes de composants (1, 2), dans laquelle les signaux de mesure du capteur rotatif (1.8, 2.8) peuvent être envoyés au circuit (1.6).

9. Procédé de surveillance de l'état d'une unité de bague collectrice, qui est configurée selon la revendication 1, dans lequel
- on transmet un courant électrique (S) du premier raccord (10) du premier groupe de composants (1) au second raccord électrique (20) du second groupe de composants (2), dans lequel
- on transmet une tension électrique (U) par la seconde piste (S2) au circuit (1.6), et
- on effectue une surveillance d'état par le circuit (1.6), de telle manière que l'on détermine une valeur caractéristique de l'état de l'unité de bague collectrice en se basant sur la tension (U).

10. Procédé selon la revendication 9, dans lequel
- on détermine la hauteur du courant (S) et on envoie cette valeur de mesure au circuit (1.6) et
- on effectue une surveillance d'état par le circuit (1.6), de telle manière que l'on détermine une valeur caractéristique de l'état de l'unité de bague collectrice en se basant sur la tension (U) et sur le courant (S).

11. Procédé selon la revendication 9 ou 10, dans lequel on ne conduit que temporairement le courant de contrôle (I) par la première piste (S1) et la seconde piste (S2).

12. Procédé selon la revendication 9, 10 ou 11, dans lequel on ne conduit que temporairement le courant de contrôle (I) par la première piste (S1) et la seconde piste (S2) et on détermine pendant ce temps la hauteur du courant (S).

13. Procédé de surveillance de l'état d'une unité de bague collectrice selon l'une quelconque des revendications 9 à 12, dans lequel
- on mesure une position de rotation relative entre le premier et le second groupes de composants (1, 2) ou le nombre de révolutions entre le premier et le second groupes de composants (1, 2) et on envoie au circuit (1.6) les signaux de mesure qui en résultent, et
- on effectue une surveillance d'état par le circuit (1.6), de telle manière que l'on détermine une valeur caractéristique de l'état de l'unité de bague collectrice en se basant sur la tension (U) et sur les signaux de mesure.

14. Procédé de surveillance de l'état d'une unité de bague collectrice selon l'une quelconque des revendications 9 à 13, dans lequel
- on mesure une humidité relative et on envoie au circuit (1.6) la valeur de mesure de l'humidité relative, et
- on effectue une surveillance d'état par le circuit (1.6), de telle manière que l'on détermine une valeur caractéristique de l'état de l'unité de bague collectrice en se basant sur la tension (U) et sur l'humidité relative.

15. Procédé de surveillance de l'état d'une unité de bague collectrice selon l'une quelconque des revendications 9 à 14, dans lequel on mémorise dans l'unité de bague collectrice une valeur limite, qui est comparée par le circuit (1.6) avec une valeur caractéristique de l'état de l'unité de bague collectrice, dans lequel une alerte est émise par l'unité de bague collectrice en cas de dépassement de la valeur limite.
